# EUROPEAN PATENT SPECIFICATION

(11) **EP 2 608 791 B1**
(45) Date of publication and mention of the grant of the patent: **18.05.2016**
(21) Application number: 10778745.9
(22) Date of filing: 07.10.2010
(51) Int. Cl.: A61K 31/506, C07D 401/04, C07D 295/155, A61P 35/00

(54) **A PROCESS FOR THE PREPARATION OF IMATINIB BASE**
VERFAHREN ZUR HERSTELLUNG EINER IMATINIB-BASE
PROCÉDÉ POUR LA PRÉPARATION D'IMATINIB BASE

(30) Priority: 23.08.2010 TR 201007005
(43) Date of publication of application: 03.07.2013
(73) Proprietor: Mustafa Nevzat Ilac Sanayii A.S., 34349 Gayrettepe (TR)
(72) Inventor: GÜNDÜZI, Halit, Ekerpinar-Cayirova/Kocaell 41480 (TR); ÖZLÜ, Yusuf, Ekerpinar-Cayirova/Kocaell 41480 (TR); YALCIN, Serkan, Ekerpinar-Cayirova/Kocaell 41480 (TR)
(74) Representative: Kodron, Felix
(86) International application number: PCT/TR2010/000198
(87) International publication number: WO 2012/026897

(56) References cited:
- WO-A1-2008/136010
- WO-A2-2004/074502

## Description

### Field of invention:

The present invention describes a new environmentally friendly high yielding process for preparation of imatinib base of Formula (I), without use of an organic solvent and where the obtained yields are 10-15% higher than the previously published methods.

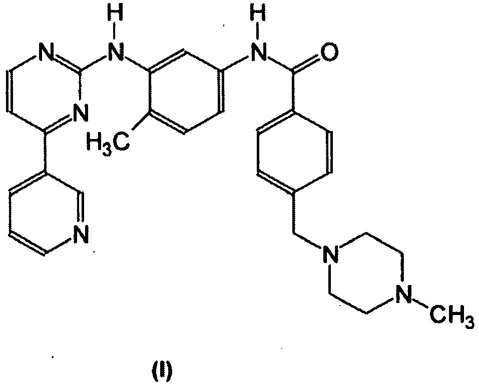

### Back ground of the invention:

The chemical structure of imatinib molecule, that is also known as 4-[(4-methylpiperazin-1-yl)methyl]-N-[4-methyl-3-[(4-pyridin-3-ylpyrimidin-2-yl)amino]phenyl]benzamide is represented in Formula (I).

Imatinib molecule is applied as a mesylate salt having the Formula (1a) in the treatment of Chronic Myeloid Leukemia where it acts as tyrosine kinase inhibitor and is effective in the treatment of Philedelphia Chromosome positive leukemia, gastrointestinal stromal tumors and a number of other malignancies.

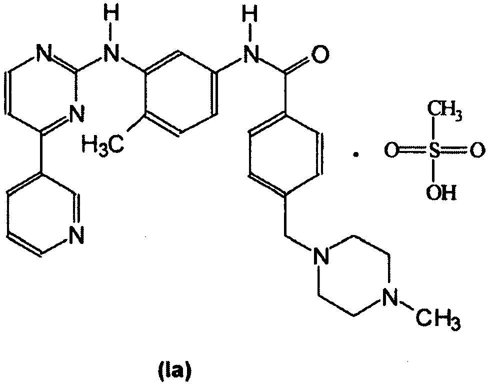

The first synthesis of Imatinib mesylate is described in WO 99/03854. According to this patent, the imatinib mesylate is prepared from imatinib base, and the preparation of latter being to referred to EP 0564409, that is the first patent for the preparation of imatinib base.

The prior art of synthesis in EP 0564409 describes the following route as illustrated in Scheme- 1. According to this patent, a solution of -(5-Amino-2-methylphenyl)-4-(3-piridinyl)-2-pirimidine amine of the Formula (II) and 4-(4-methylpiperazine-1-yl-methyl)benzoyl chloride of the formula (III) taken in pyridine are stirred under nitrogen at room temperature for 23 hours. The crude product imatinib of Formula (I) is purified by column chromatography. After implementing this process described in this patent the following are difficulties encountered and draw backs noticed:
- the yield of imatinib of the Formula (I) is low (50%) making this process non-economical
- the purity of the product is only 97%
- column chromatography is necessary for the purification of the imatinib of the Formula (I) and column chromatography technique becomes unpractical on industrial scale
- usage of obnoxious and foul smelling pyridine as a solvent makes this process to be abandoned on commercial scale

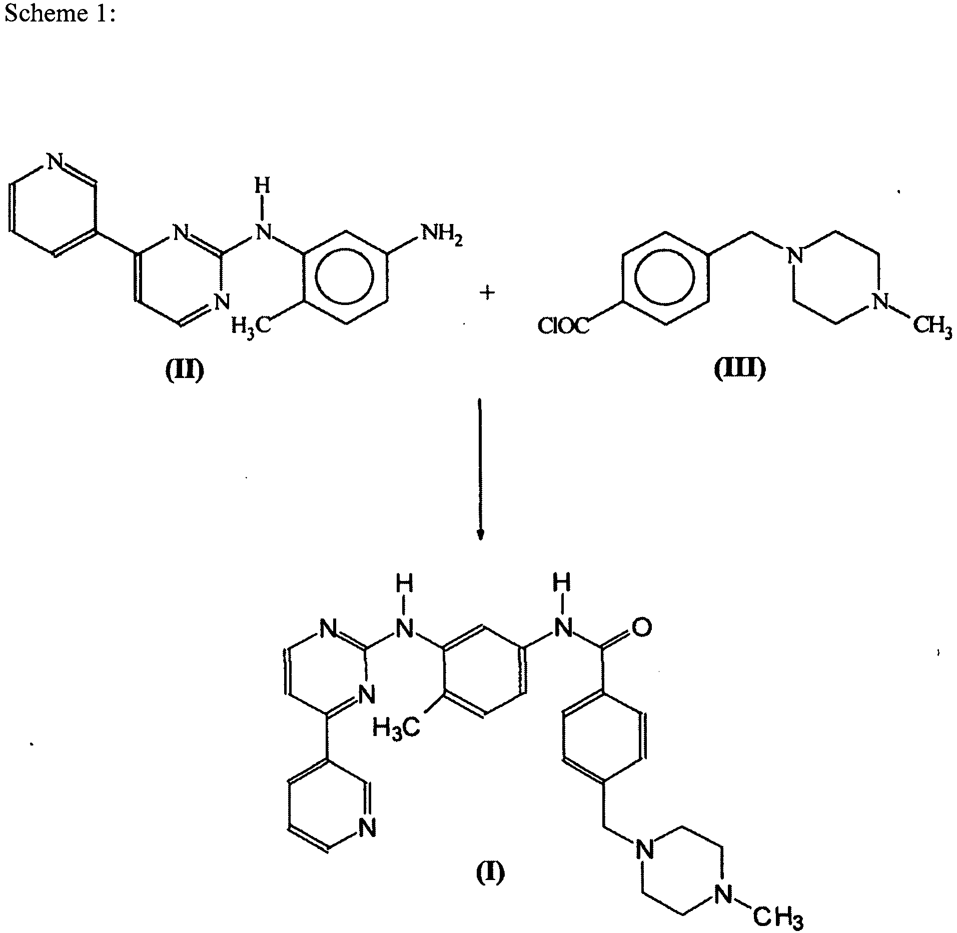

A similar process is described in WO 2004/074502 patent. This methodology also uses the amine compound of the Formula (II) and the chloride compound of the Formula (III), as represented in Scheme- 1, as starting materials. Only difference from the previous methodology is the use of N,N-dimethylformamide (DMF) as organic solvent instead of pyridine. This study reveals that the process of the reaction proceeds over a mechanism to form imatinib hydrochloride which is the isolated as imatinib hydrochloride salt from the reaction mixture. This methodology is not easy to perform in bulk scale due to usage of high boiling solvents in large volumes. Usage of high boiling and very polar solvents such as DMF or N,N-dimethylacetamide (DMACT) causes problems during the distillation process of solvent recovery. Additionally, the need for an inert atmosphere and high reaction temperatures (70°C) is required for obtaining the final product in good yields. For the reasons mentioned above, the application of such a process in commercial scale is not preferred and needs to be improved for cost effectiveness.

Scheme 2, illustrates another production methodology for imatinib base of the Formula (I) as described in patent application WO 03/066613. In this route, imatinib base of the Formula (I) is produced in two routes; in the first route, condensation reaction of of 4-(3-piridinyl)-2-pirimidine amine of the Formula (IV) with N-(3-bromo-4-methyllfenyl)-4-(4-metilpiperazine-1-yl-methyl)benzamide of the formula (V) is realized in the presence of a palladium catalyst in xylene under argon atmosphere, and in the second route, 3-amino-1-pyridin-3-yl-propenone of the formula (VI) and N-(3-guanidino-4-methylphenyl)-4-(4-methylpiperazin-1-ylmethyl)-benzamide of the Formula (VII) is reacted in n-butanol at 150°C for 5 hours.

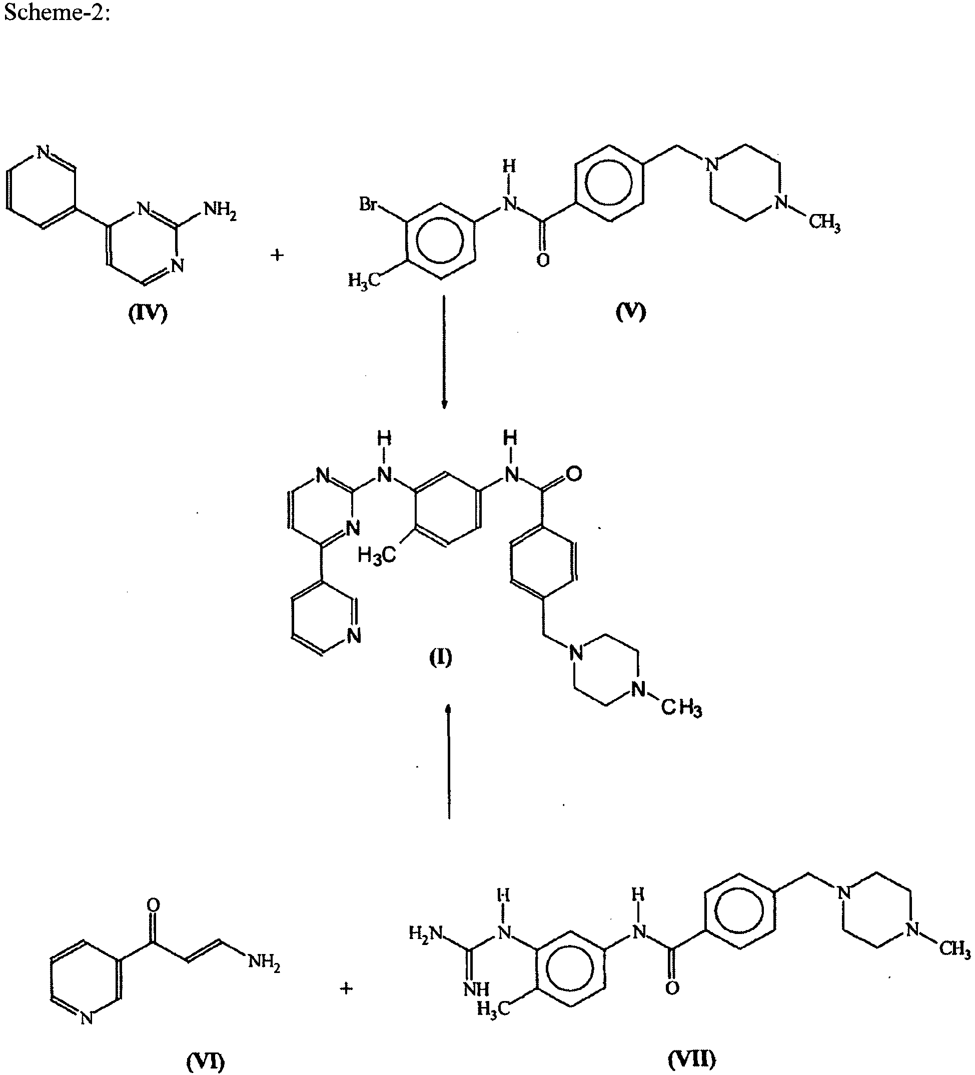

However, these described methods are not also feasible for industrial scale productions. Usage of palladium catalyst is expensive, and toxic. Also usage of harsh conditions such as high temperatures and inert atmosphere make this methodology more expensive and problematic for bulk scale productions.

Another prior art as described in patent applications of US2008/0194819 and WO 2004/108699 is presented in Scheme-3. In these patents, 4-methyl-N-3-(4-pyridine-3-yl-pyrimidine-2-yl)benzen-1,3-diamine of the Formula (II) is taken into a condensation reaction with 4-chloromethylbenzoyl chloride of the Formula (VIII) to give 4-chloromethyl-N-{4-methyl-3-[(4-pyridine-3-yl)pyrimidine-2-yl-amino]phenyl}benzamide of the Formula (IX) that is again reacted with N-methylpiperazine to give the final imatinib of the Formula (I) as final product. The use of organic bases such as triethyl amine and piperidine as well as organic solvents such as chloroform and DMF are the major draw backs of the method. These reactants may possible be trapped in the imatinib base final product and cause the formation of unwanted impurities. The proposed method is also not suitable for large scale industrial production due to the risks of the applied organic solvents on the occupational health, environmental safety issues as well as problematic recovery procedures.

Imatinib base is the precursor of imatinib salts, especially the therapeutic imatinib mesylate. As such, there is a need for imatinib base of high quality which may be used as precursor in the highly pure imatinib mesylate for therapeutic application.

It is observed that mesylate salt of imatinib, when prepared from imatinib of lower purity do not meet the pharmaceutically acceptable quality. There is therefore still a need for a process which can be applied industrially to prepare high quality imatinib base of the Formula (I).

On the other hand, high quality imatinib base can only be produced if the problems arising from reagents and solvents used in the production can be eliminated. Therefore the usage of reagents and solvents harmful to health should be avoided and new processes should be safe from the point of environmental protection.

When the above mentioned prior art applied in the industrial scale they do not fulfill one or other of the above requirements. Prior art methods mainly suffer because they are not economically feasible and the obtained final products are not always in the required quality. For these reasons, there is a need for new processes that are applicable in industrial scale; producing higher yields, being economical, and harmless to the environment and occupational health.

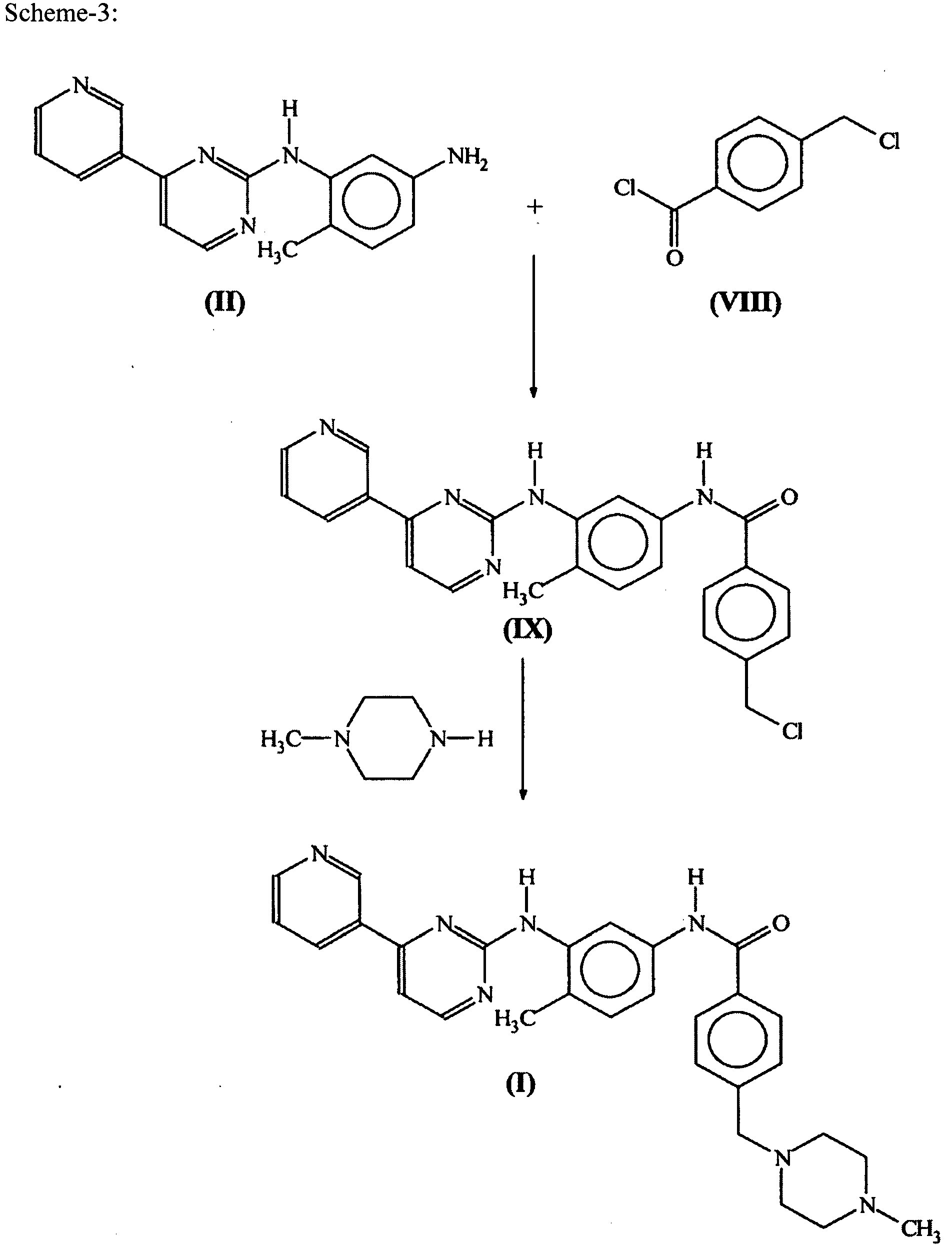

Therefore, we directed our efforts to develop an improved process for the preparation of imatinib base of the Formula (I) with the aim to provide a new environmentally protective, safe, economical and industrially applicable process which is devoid of the insufficiencies of the prior art methods.

### Summary of invention:

The main object of the present invention is to provide an improved process for the preparation of imatinib base in an economical and environmentally protective way.

Another object of the present invention is to provide highly pure imatinib base which can be used as precursor in the synthesis of therapeutically active imatinib salts.

Accordingly in the present invention highly pure imatinib base is prepared by
i. synthesizing imatinib hydrochloride of the Formula (Ic) by condensation of N-(5-Amino-2-methylphenyl)-4-(3-piridinyl)-2-pirimidine amine of the Formula (II) and 4-(4-methylpiperazine-1-yl-methyl)benzoyl chloride dihydrochloride of the Formula (Ilia) in water
ii. precipitating the resulting imatinib hydrochloride of the Formula (Ic) from the aqueous reaction solvent by addition of inorganic base
iii. purifying the imatinib base by crystallization in alcohols an isolating highly pure imatinib

### Detailed description of the invention:

The present invention describes a new method developed for the production of imatinib base. The developed method is based on the condensation reaction of 4-(4-methylpiperazine-1-yl-methyl)benzoyl chloride dihydrochloride of the Formula (Ilia) and N-(5-Amino-2-methylphenyl)-4-(3-piridinyl)-2-pirimidine amine of the Formula (II) in completely organic solvent free environment, in water to give imatinib base. The synthesis of imatinib mesylate of the Formula (1a) can also be performed using the produced imatinib base with the described in this patent without further purification. Production scheme of the newly developed method is shown in Scheme 4

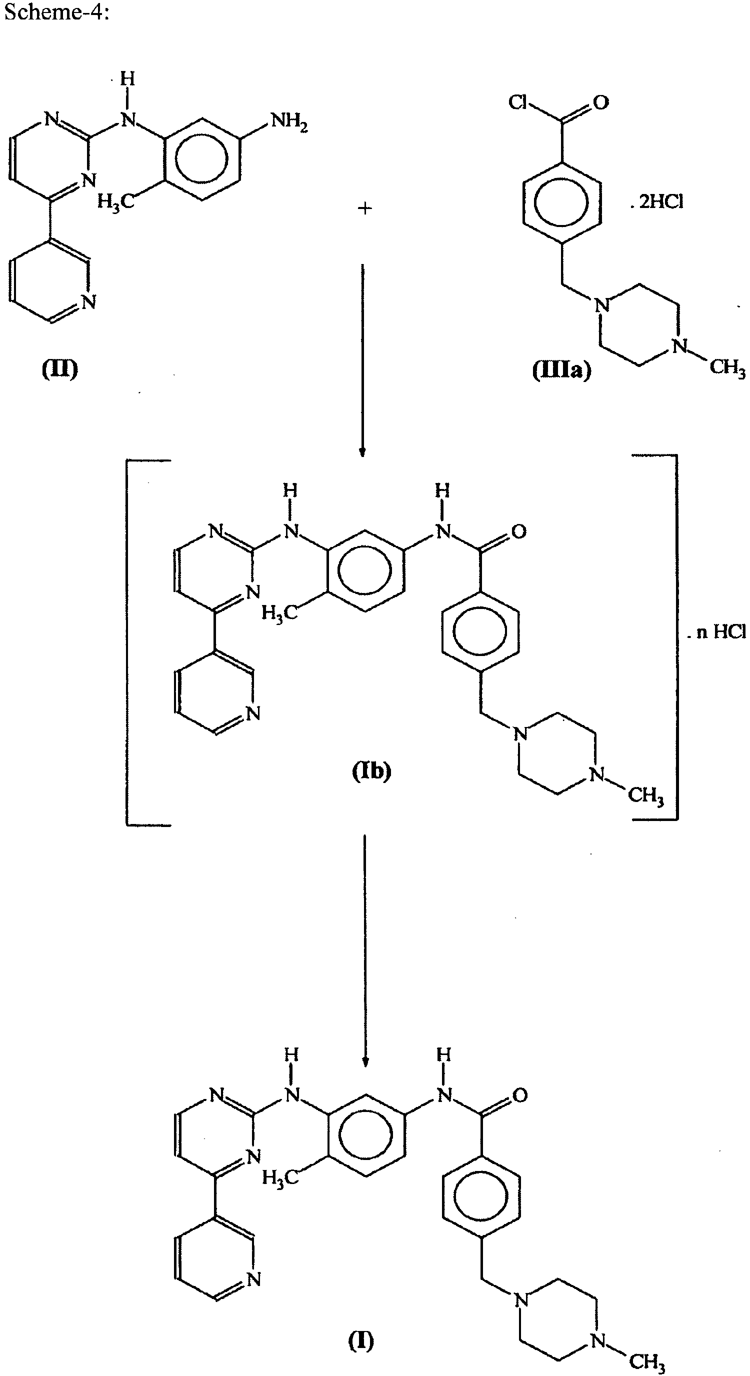

During the course of reaction the resulting imatinib molecule is in the form of hydrochloride salt of the Formula (Ic) that has a high solubility in water. The hydrochloric acid molecules required to form hydrochloride salt of imatinib are supplied by starting material of 4-(4-methylpiperazine-1-yl-methyl)benzoyl chloride di-hydrochloride of the Formula (Ilia) and also from the hydrochloric acid molecules that is formed as a by-product during the reaction. The produced imatinib hydrochloride of the Formula (Ic) is not isolated from the reaction medium, instead is directly converted to imatinib base of the Formula (I). At the final step of the reaction, pH value of the reaction mixture is increased by adding organic or inorganic bases thus allowing the precipitation of imatinib base.

The reaction course is followed up by an HPLC method until, N-(5-Amino-2-methylphenyl)-4-(3-piridinyl)-2-pirimidine amine of the Formula (II) is totally consumed in the reaction medium. Although, N-(5-Amino-2-methylphenyl)-4-(3-piridinyl)-2-pirimidine amine of the Formula (II) is poorly soluble in water, it was found that, during the course of the reaction it is gets soluble due to acidic reaction conditions. The completion of the reaction is determined by total disappearance of N-(5- Amino-2-methylphenyl)-4-(3-piridinyl)-2-pirimidine amine of the Formula (II) by HPLC. Furthermore, un-reacted N-(5-Amino-2-methylphenyl)-4-(3-piridinyl)-2-pirimidine amine of the Formula (II) can easily be removed from the reaction environment by filtration after the reaction as it is not soluble in water.

On the other hand, it was found that, 4-(4-methylpiperazine-1-yl-methyl)benzoyl chloride di-hydrochloride of the formula (Ila) undergoes a degradation process in water to give the corresponding acid compound, namely, 4-(4-methylpiperazine-1-yl-methyl)benzoic acid. In order to reduce the rate of degradation of this material of the Formula (Ilia) the reaction is performed at cold temperatures and/or feeding of this reagent is performed in portions.

The reactant quantities can be in the stoichiometric ratios. However, in order to obtain better yields, 4-(4-methylpiperazine-1-yl-methyl)benzoyl chloride di-hydrochloride of the Formula (Ilia) may be used in excess. Based on the N-(5-Amino-2-methylphenyl)- 4-(3-piridinyl)-2-pirimidine amine of the Formula (II) amount the acid chloride of the Formula (Ilia) may be between 1.05 to 2.5 mole equivalents in excess. Preferably acid chloride amount is in 1.25 to 2.25 mole equivalents. It was found that usage of 1.5 to 2 mole equivalents of the acid chloride of the Formula (Ilia) is necessary for the highest yield.

The temperature of the reaction mixture should be kept between 0°C to 25°C. It has been observed that the degradation rate of the 4-(4-methylpiperazine-1-yl- methyl)benzoyl chloride dihydrochloride of the Formula (Ilia) increases with the increased temperature. For this reason temperature of the reaction mixture should be kept at below 10°C. For the best conversion the suitable temperature range should be between (-5°C) to 10°C. To achieve the optimized yields, the reaction temperature should be kept at between 0°C to 5°C during the reaction.

It was found that, 4-(4-methylpiperazine-1-yl-methyl)benzoyl chloride di-hydrochloride of the Formula (Ilia) can easily be converted to the undesired corresponding carboxylic acid compound, namely 4-(4-methylpiperazine-1-yl-methyl)benzoic acid, by hydrolysis reaction with water. In order to minimize this conversion, the reaction should be performed in cold and also 4-(4-methylpiperazine-1-yl-methyl)benzoyl chloride di-hydrochloride of the Formula (Ilia) should be added to the reaction mixture in portions. The amounts of the portions and addition times may vary, however preferred conditions can be set as 2-3 hours of addition with 4% to 8% of portions amount. For higher yields, three hours of adding time with 6% of portions amount was found to be optimum.

It was observed that the reaction proceeds rapidly, following the addition of the last portion of 4-(4-methylpiperazine-1-yl-methyl)benzoyl chloride di-hydrochloride of the Formula (Ilia). However, 1 to 3 hours of further stirring under cold conditions can also be performed. Favorably, 30 minutes of stirring after the addition of last portion will be adequate. Total reaction time varies between 3 to 3.5 hours.

The course of the reaction can be followed up by any techniques used in organic chemistry such as; HPLC, TLC. Preferably HPLC is used for the reaction monitoring, more specifically, total consumption of N-(5-Amino-2-methylphenyl)-4-(3-piridinyl)-2-pirimidine amineof the Formula (II) in the reaction mixture is monitored. It has been observed that extending the reaction time does not positively affect the conversion rate. When the reaction conversion is not sufficient more excess of, 4-(4-methylpiperazine-1-yl-methyl)benzoyl chloride di-hydrochloride of the Formula (Ilia) can be added portionwise to the reaction mixture.

Upon completion of the reaction, reaction mixture is filtrated over celite bed to remove any unreacted starting materials and other insoluble particles. In order to precipitate imatinib base of the Formula (I) from the reaction mixture, inorganic base is added to make the mixture basic. For the precipitation , optimum crystallization pH range is found to be 8-12 and more preferably, the preferred pH range is set as 9-10. The pH of the imatinib hydrochloride solution that is obtained after the reaction is adjusted to this range by using appropriate base. For the adjustment of pH any organic or inorganic base can be applied. Inorganic bases, such as, ammonium hydroxide, potassium hydroxide, and sodium hydroxide can be used for this purpose. In this content, sodium hydroxide is preferred for the adjustment of pH of the reaction mixture to the desired value. Sodium hydroxide can be added to the reaction mixture in solid form or as defined concentrated solution. By increasing the pH value of the reaction medium imatinib hydrochloride of the Formula (Ic) molecule is converted to imatinib base of the Formula (I) which is not soluble in water, thus leading to precipitate as solid from the reaction mixture. Obtained solid product is isolated from the solution by filtration. During the course of precipitation, the resulting solid may either be in amorphous form or in the crystalline form. It has been observed that the filtration of imatinib base which is in the amorphous form is very difficult and the quality of the obtained product was found to be poor. For this reason, during the precipitation process the crystalline form of imatinib base is preferred. In order to obtain crystalline form of imatinib base, pH adjustment and precipitation should be performed in temperatures above 25°C. The optimum temperature range is found to be between 30°C to 65 °C. Preferably, precipitation process is to be performed between 50°C to 55°C to obtain the target product in good yield and quality.

The quality of the Imatinib base produced with the developed method is found to be comparable against literature data. The potency of the imatinib base molecule produced with this method is found to be around 98%. At this stage, re-crystallization process with ethyl alcohol can be performed in order to increase purity. Ethyl alcohol re-crystallization can be performed once or twice. In our study one time re-crystallization of the imatinib base molecule was found to be satisfactory giving minimum 99% assayed product. Imatinib base product obtained after rec-crystallization is suitable for imatinib mesylate production in terms of impurities and assay, and can be used without further purification process.

The developed process presented herein displays 10%- 15 higher yields when compared to previously published processes. The major impact on the increased yield is the low water solubility of the starting material N-(5-Amino-2-methylphenyl)-4-(3-piridinyl)-2r pirimidine amine of the Formula (II) that prevents the transformation of this material to unwanted side products. Additionally, the reaction environment of the developed process is milder when compared against the literature data. In the literature, harsh reaction conditions such as, heating, prolonged reaction times or use of a catalyst are needed leading the poor yields.

Another drawback of the published methods is the use of an organic solvent as the reaction medium. Usage of organic solvents may lead some of the imatinib base to stay soluble in the reaction mixture during the precipitation process which results in some loss of material. In this context, usage of water as the reaction medium prevents the product loss during the filtration process thus leading to higher process yields.

As mentioned earlier, patent EP 0564409 uses pyridine for the synthesis of imatinib base, whereas patent application WO 2004/074502 suggests the use of DMF instead of pyridine. The usage of organic solvents in both methods may lead to the observation of these solvents in the final product as residual solvent impurities. In the prior art, the mother liquor obtained from the filtration process is usually a mixture of organic solvent/ water that may contain final product imatinib base and as well as starting materials. The separation and elimination of these waste material causes increased economic burden to production costs. With the present developed method, the mother liquor obtained from the filtration process of the final product is only water and can be treated more easily and in a cost efficient way. The amount of imatinib base final product in this aqueous liquor is in trace amounts and therefore the waste water is not contaminated with imatinib. In a similar manner, the ethyl alcohol used in the re- crystallization process can be easily recovered with simple distillation to be used again. For these reasons the developed method can be considered as an environmentally friendly process.

The starting materials 4-(4-methylpiperazine-1-yl-methyl)benzoyl chloride dihydrochloride of the Formula (Ilia) and N-(5-Amino-2-methylphenyl)-4-(3-piridinyl)-2-pirimidine amine of the Formula (II) can easily be produced according to literature data or can be obtained commercially.

In conclusion, the developed method displays the below listed advantages when compared against the previously published methods:
- Ease of industrial applicability
- Increased yield of the final product
- Lower costs on the solvent usage and recovery processes
- Increased quality of the final product
- More secure process with harmless solvent usage
- Environmentally friendly and protective

The final imatinib base product obtained with the process described in here, can be directly converted to other imatinib salts especially to imatinib mesylate salt which is widely used as a final drug form to be served for human health.

The detailed experimental procedure of the described process is given below. These details are given as an experimental insight and do not contain whole of the invention.

### Examples:

### Example 1: Synthesis of Imatinib base

N-(5-Amino-2-methylphenyl)-4-(3-piridinyl)-2-pirimidine amine (15 g ; 54,09 mmole) and process water (350 g) are taken into round bottom flask. The temperature of the reaction mixture is adjusted to between 0°C to 5°C. At this temperature, 4-(4-methylpiperazine-1-yl-methyl)benzoyl chloride di-hydrochloride (34 g ; 104,41 mmol) is added to the reaction flask in portions of 2 g in every 10 minutes for 17 times. The reaction is followed by HPLC and the reaction is carried out until the N-(5-Amino-2-methylphenyl)-4-(3-piridinyl)-2-pirimidine amine is completely consumed. Activated charcoal (3.5 g) is added to reaction and stirred for 15 minutes. The reaction mixture is filtered over a bed of celite. Filter bed is washed with process water (30 g). The temperature of the obtained solution is increased between 50°C to 55°C. At this temperature 20% NaOH is added until obtaining a pH value between 9 to 9.5. Following the stabilization of pH value, the formed precipitate is filtered under vacuum. Isolated product is washed with additional process water (30 g) and kept under vacuum for an additional 30 minutes. Product is dried in a vacuum oven at 60°C.

Yield 26,5 g (53,69 mmol; 99,26%)

### Example 2: Purification of Imatinib base

Crude Imatinib base (26,5 g ; 53,69 mmole) and ethyl alcohol (320 g) is taken in a round bottom flask. The temperature of the reaction mixture is set to the reflux temperature. At this temperature crude imatinib base is completely dissolved. Following the complete dissolution reaction temperature is set between 55°C to 60°C. To this solution activated charcoal is added (3.5 g) and stirred for 15 minutes. Reaction mixture is filtrated over celite bed while hot and filter is washed with hot ethyl alcohol (20 g). The temperature of the reaction mixture is allowed to reduce while stirring for 2 hours. Below 50°C a precipitate forms. In order to increase precipitation amount reaction mixture is cooled between (-5°C) to 0°C and stirred for five hours at this temperature. The product is obtained via vacuum filtration followed by cold ethanol washing. Isolated product is kept under vacuum for 30 minutes followed by drying at 60°C.

Yield: 23 g (46,60 mmol; 86,79%)

## Claims

1. A process for preparing imatinib base, wherein the process is **characterized by**
i. a reaction of 4-(4-methylpiperazine-1-yl-methyl)benzoyl chloride dihydrochloride and N-(5-Amino-2-methylphenyl)-4-(3-piridinyl)-2-pirimidine amine in water to obtain imatinib hydrochloride, wherein the reaction is performed at a temperature of -5°C to 10°C and 4-(4-methylpiperazine-1-yl-methyl)benzoyl chloride dihydrochloride is added to the reaction mixture in portions of 4% to 8% over a period of 2 to 3 hours;
ii. a crystallization step by pH adjustment with alkaline metal hydroxides to obtain imatinib base;
iii. a recrystallization step of the resulting product in a suitable solvent.

2. The process of claim 1, wherein the reaction is performed at a temperature of 0°C to 5°C.

3. The process of claim 1, wherein 4-(4-methylpiperazine-1-yl-methyl)benzoyl chloride dihydrochloride is added to the reaction mixture in portion of 6% over a period of 3 hours.

4. The process of claim 1, wherein the obtained imatinib hydrochloride is directly converted to imatinib base without further isolation.

5. The process of claim 1, wherein imatinib base is crystallized in water environment by pH adjustment.

6. The process of claim 5, wherein pH adjustment is performed with sodium hydroxide.

7. The process of claim 1, wherein imatinib base is recrystallized using a suitable alcohol.

## Patentansprüche

1. Verfahren zur Herstellung von Imatinib-Base, wobei das Verfahren **gekennzeichnet ist durch**
i. eine Reaktion von 4-(4-Methylpiperazin-1-ylmethyl)benzoylchloriddihydrochlorid und N-(5-Amino-2-methylphenyl)-4-(3-pyridinyl)-2-pyrimidinamin in Wasser unter Erhalt von Imatinibhydrochlorid, wobei die Reaktion bei einer Temperatur von -5°C bis 10°C durchgeführt wird und 4-(4-Methylpiperazin-1-ylmethyl)benzoylchloriddihydrochlorid der Reaktionsmischung in Portionen von 4% bis 8% über einen Zeitraum von 2 bis 3 Stunden zugesetzt wird,
ii. einen Kristallisationsschritt **durch** Einstellen des pH-Wertes mit Alkalihydroxiden unter Erhalt von Imatinib-Base,
iii. einen Umkristallisationsschritt des erhaltenen Produkts in einem geeigneten Lösungsmittel.

2. Verfahren nach Anspruch 1, wobei die Reaktion bei einer Temperatur von 0°C bis 5°C durchgeführt wird.

3. Verfahren nach Anspruch 1, wobei 4-(4-Methylpiperazin-1-ylmethyl)benzoylchloriddihydrochlorid der Reaktionsmischung in Portionen von 6% über einen Zeitraum von 3 Stunden zugesetzt wird.

4. Verfahren nach Anspruch 1, wobei das erhaltene Imatinibhydrochlorid ohne weiteres Isolieren direkt in Imatinib-Base überführt wird.

5. Verfahren nach Anspruch 1, wobei Imatinib-Base in wässriger Umgebung durch Einstellen des pH-Wertes kristallisiert wird.

6. Verfahren nach Anspruch 5, wobei das Einstellen des pH-Wertes mit Natriumhydroxid erfolgt.

7. Verfahren nach Anspruch 1, wobei Imatinib-Base unter Verwendung eines geeigneten Alkohols umkristallisiert wird.

## Revendications

1. Procédé de préparation d'imatinib base, où le procédé est **caractérisé par**
i. une réaction de dichlorhydrate de chlorure de 4-(4-méthylpipérazine-1-yl-méthyl)benzoyle et de N-(5-amino-2-méthylphényl)-4-(3-piridinyl)-2-pirimidinamine dans de l'eau pour obtenir du chlorhydrate d'imatinib, où la réaction est conduite à une température de -5 °C à 10 °C et du dichlorhydrate de chlorure de 4-(4-méthylpipérazine-1-yl-méthyl)benzoyle est ajouté au mélange de réaction en portions de 4 % à 8 % sur une période de 2 à 3 heures ;
ii. une étape de cristallisation par ajustement du pH avec des hydroxydes de métal alcalin pour obtenir de l'imatinib base ;
iii. une étape de recristallisation du produit résultant dans un solvant adapté.

2. Procédé de la revendication 1, dans lequel la réaction est conduite à une température de 0 °C à 5 °C.

3. Procédé de la revendication 1, dans lequel le dichlorhydrate de chlorure de 4-(4-méthylpipérazine-1-yl-méthyl)benzoyle est ajouté au mélange de réaction en portions de 6 % sur une période de 3 heures.

4. Procédé de la revendication 1, dans lequel le chlorhydrate d'imatinib obtenu est directement converti en imatinib base sans isolement supplémentaire.

5. Procédé de la revendication 1, dans lequel l'imatinib base est cristallisé dans un environnement aqueux par ajustement de pH.

6. Procédé de la revendication 5, dans lequel l'ajustement du pH est effectué avec de l'hydroxyde de sodium.

7. Procédé de la revendication 1, dans lequel l'imatinib base est recristallisé en utilisant un alcool adapté.
